(19) Europäisches Patentamt
European Patent Office
Office européen des brevets

(11) Veröffentlichungsnummer: **0 098 447**
**B1**

# (12) EUROPÄISCHE PATENTSCHRIFT

(45) Veröffentlichungstag der Patentschrift: **07.01.88**

(51) Int. Cl.⁴: **C 07 D 317/30** // C07D495/04, C07D333/00, C07D235/00

(21) Anmeldenummer: **83106024.9**

(22) Anmeldetag: **21.06.83**

(54) **Verfahren zur Herstellung von (6R,7S,8R)-6,9-Dihydroxy-7,8-isopropylidendioxy-2,4-nona-diensäurealkylestern und deren Verwendung zur Herstellung von d-Biotin.**

(30) Priorität: **01.07.82 DE 3224511**

(43) Veröffentlichungstag der Anmeldung: **18.01.84 Patentblatt 84/3**

(45) Bekanntmachung des Hinweises auf die Patenterteilung: **07.01.88 Patentblatt 88/1**

(84) Benannte Vertragsstaaten: **CH DE FR GB LI NL**

(56) Entgegenhaltungen:
**DE - C - 3 122 562**

**CHEMICAL ABSTRACTS Band 94, Nr. 15, 13. April 1981Columbus, Ohio, USA; F. VOGEL et al. "(+)-Biotin from D-arabinose" Seite 688, Spalte 2, Abstract Nr. 121 407 n & Liebigs Ann. Chem. Band 1980, Nr. 12, Seiten 1972-1977 (Kat. D)**

(73) Patentinhaber: **BASF Aktiengesellschaft, Carl-Bosch-Strasse 38, D-6700 Ludwigshafen (DE)**

(72) Erfinder: **Schmidt, Richard, Dr., Grossherzog-Friedrich-Strasse 11, D-7750 Konstanz 16 (DE)**
Erfinder: **Maier, Martin, Egelseeweg 13, D-7750 Konstanz 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



EP 0 098 447 B1

**Beschreibung**

Die Erfindung betrifft ein Verfahren zur Herstellung von (6R,7S,8R)-6,9-Dihydroxy-7,8-isopropylidendioxy-2,4-nonadiensäurealkylestern durch Umsetzen von 3,4-O-Isopropyliden-D-arabinose mit 3-Carbomethoxy-propen-(2)-yliden-(1)-triphenylphosphoren sowie dessen Verwendung zur Herstellung von d-Biotin der Formel

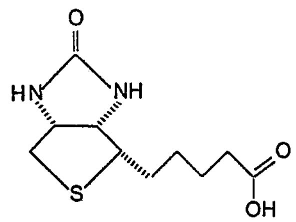

Biotin hat wie alle Vitamine in den letzten Jahren zunehmend an Bedeutung gewonnen. Da nur die d-Form des Biotins physiologisch wirksam ist, hat es nicht an Versuchen gefehlt, ein wirtschaftliches Verfahren zur Herstellung von d-Biotin zu finden. Zur Vermeidung der im allgemeinen recht aufwendigen Racemattrennungen ist man bemüht, die gewünschten chiralen Verbindungen durch stereospezifische Umwandlungen bequem verfügbarer chiraler Vorläufer herzustellen. So veröffentlichten japanische Autoren Biotinsynthesen, bei denen das Grundgerüst dieses Vitamins aus natürlich vorkommenden Hexosen aufgebaut wird (vgl. z.B. Ohrui et al., Tetrahedron Lett. 1975, 2765). Nachteilig an diesem an sich vergleichsweise wirtschaftlichen Verfahren ist die Notwendigkeit, das ursprüngliche Kohlenstoffgerüst um 1 C-Atom zu verkürzen.

Es wurde daher versucht, von leicht zugänglichen Pentosen, speziell von D-Arabinose, auszugehen.

Will man aus D-Arabinose (d)-Biotin herstellen, so muss man in 1-Stellung eine $C_4$-Kette anknüpfen, zwischen 2 und 5 einen Thiophanring bilden und in 3- und 4-Stellung N-Atome einführen. Die N-Atome werden zweckmässigerweise am Ende des Syntheseweges eingeführt, deshalb werden zu Beginn die 3- und 4-Stellung geschützt.

So haben F. Vogel et al. (vgl. Liebigs Ann. Chem. 1980, 1972–77) versucht, durch Umsetzen des leicht aus Arabinose zugänglichen 3,4-Isopropyliden-Derivats der Formel II

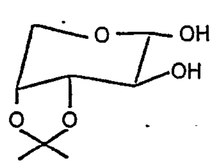

(II)

mit 3-Carbomethoxy-propen-(2)-yliden-(1)-triphenylphosphoran der Formel IIIa

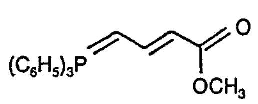

(IIIa)

(6R,7S,8R)-6,9-Dihydroxy-7,8-isopropylidendioxy-2,4-nonadiensäurealkylestern der Formel Ia

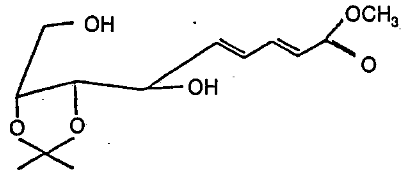

(Ia)

herzustellen, welches sich nach katalytischer Hydrierung zu der entsprechenden gesättigten Verbindung nach dem Verfahren von Ohrui et al. (vgl. Tetrahedron Lett. (1975) 3657 in 8 Stufen in (d)-Biotin überführen liess. Diese Versuche ergaben jedoch nach Angaben der Autoren wenig ermutigende Resultate. Mit dem in 2-Stellung benzylierten II erhielten sie zwar einen vollständigen Umsatz, jedoch ein sehr komplexes Reaktionsgemisch. Auch mit dem in 2-Stellung benzoylierten II gelang die Kettenverlängerung der Arabinose in 1-Stellung nur in einer Ausbeute von etwa 13%.

Im Rahmen seiner Diplomarbeit untersuchte M. Maier (vgl. Diplomarbeit, Univ. Konstanz, 1981) erneut die Umsetzung von I mit III.

Er arbeitete in Dimethylformamid/$CH_2Cl_2$ (1:1), Dimethylsulfoxid und Dimethoxyethan bei Temperaturen von 85 bzw. 90 °C und in Gegenwart katalytischer Mengen Benzoesäure. Die von ihm erzielten Ausbeuten an I lagen zwischen 33 und 39% der Theorie. Auf Seite 19, Zeilen 1–2 seiner Diplomarbeit schreibt er wörtlich über diese Umsetzung: «Die Ausbeuten konnten auch durch Variation der Reaktionsbedingungen nicht wesentlich verbessert werden». Ausbeuten in dieser Grössenordnung sind jedoch für ein technisches Verfahren nicht ausreichend.

In der nicht vorveröffentlichten Europäischen Patentschrift EP-A-066 753 wurde bereits die Umsetzung von Isopropylidenarabinose mit einem Phosphoran in cycloaliphatischen Äthern (THF und Dioxan) sowie Halogenkohlenwasserstoffen (Dichlorethan) bei erhöhter Temperatur beschrieben. Die hierbei erzielten Ausbeuten von 53 bzw. 62,8% der Theorie sind jedoch unbefriedigend für ein technisches Verfahren.

Es war daher die Aufgabe der Erfindung, ein Verfahren zu entwickeln, mit dessen Hilfe das Biotin-Zwischenprodukt I ausgehend von der leicht zugänglichen D-Arabinose auf einfache Weise und in guten Ausbeuten erhalten werden kann.

Es wurde überraschenderweise gefunden, dass man I durch Umsetzen von I mit III in recht guten Ausbeuten erhalten kann, wenn man die Umsetzung in Toluol bei Temperaturen von 80 bis 115 °C durchführt.

Gegenstand der Erfindung ist dementsprechend ein Verfahren zur Herstellung von
(6R,7S,8R)-6,9-Dihydroxy-7,8-isopropylidendioxy-2,4-nonadien-säurealkylestern
der allgemeinen Formel I

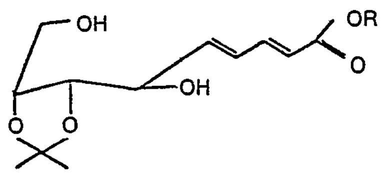

(I)

in der R für einen Alkylrest mit 1 bis 4 C-Atomen, vorzugsweise den Methylrest steht, durch Umsetzen von 3,4-O-Isopropyliden-D-arabinose der Formel II

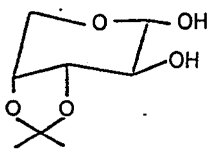

(II)

mit 3-Carboalkoxy-propen-(2)-yliden-(1)-triphenylphosphoran der allgemeinen Formel III

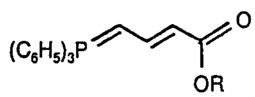

(III)

in der R die obige Bedeutung aufweist, in einem Lösungsmittel bei erhöhter Temperatur, das dadurch gekennzeichnet ist, dass man die Umsetzung in Toluol bei Temperaturen von 80 bis 115 °C durchführt.

Es war sehr überraschend, dass unter den erfindungsgemässen Reaktionsbedingungen bei der Umsetzung von II mit III die Bildung von unerwünschten Nebenprodukten, insbesondere die Bildung von cyclischen Verbindungen durch intramolekulare Michael-Addition der freien Hydroxylgruppe an das $\alpha,\beta,\sigma,\lambda$-ungesättigte System, wie sie Vogel et al. (loc. cit.) bei der Umsetzung von in 2-Stellung benzoyliertem II in grossem Ausmass beobachten mussten, trotz Einsatz von D-Arabinose mit in 1- und 2-Stellung ungeschützten Hydroxylgruppen weitgehend unterdrückt und dadurch die für die Herstellung von d-Biotin benötigten Verbindungen der allgemeinen Formel I in für Umsetzungen mit derart reaktionsfreudigen Reaktanden ausserordentlich guten Ausbeuten erhalten wurden.

Die als Ausgangsverbindung benötigte 3,4-O-Isopropyliden-D-arabinose II lässt sich durch kinetisch kontrollierte Reaktion mit Isopropenylether oder 2,2-Dimethoxy-propan in Gegenwart katalytischer Mengen p-Toluolsulfonsäure in Ausbeuten von etwa 83% herstellen [vgl. J. Gelas und D. Horton, Carbohydr. Res. 45, 181 (1975); M. Kiso und A. Hasegawa, Carbohydr. Res. 52, 95 (1976)].

Das Ylid III kann aus $\sigma$-Brom-crotonsäure-alkylestern mit Triphenylphosphin in Toluol und Umsetzung des erhaltenen Phosphoniumsalzes mit NaOH erhalten werden.

Die dünnschichtchromatographische Untersuchung von Reaktionsansätzen zeigte dass bei der Umsetzung in cycloaliphatischen Ethern mehr Nebenprodukte gebildet werden als bei der Umsetzung in Toluol.

Zur Durchführung des Verfahrens löst man im allgemeinen fein gepulverte und unter stark vermindertem Druck getrocknete 3,4-O-Isopropyliden-D-arabinose bei Raumtemperatur in dem Lösungsmittel, fügt unter einer Schutzgasatmosphäre 3-Carboalkoxy-propen-(2)-yliden-(1)-triphenylphosphoran zu und erhitzt das Reaktionsgemisch unter Schutzgasatmosphäre und unter Feuchtigkeitsausschluss in einem mit Rückflusskühler versehenen Reaktionsgefäss.

Der Fortgang der Reaktion kann durch DC kleiner Proben verfolgt werden. Die DC erfolgte im allgemeinen an Kieselgel mit reinem Essigsäureethylester als Laufmittel.

Die Aufarbeitung des Reaktionsgemisches erfolgt in üblicher Weise durch Abkühlen, Einengen der Lösung unter vermindertem Druck, Aufnehmen in Essigsäureethylester, Abfiltrieren von sich abscheidendem Phosphinoxid und chromatographische Reinigung des so erhaltenen Extraktes an Kieselgel. Beim Vorliegen von relativ reinen Extrakten, wie sie beim Arbeiten in Toluol anfallen, genügt es, mit einer kurzen Kieselgelsäule den im Extrakt enthaltenen Überschuss an Wittig-Reagenz zu entfernen.

Bei der erfindungsgemässen Umsetzung erhält man beispielsweise den (6R,7S,8R)-6,9-Dihydroxy-7,8-isopropylidendioxy-2,4-nonadiensäuremethylester in Form eines EE/EZ-Isomerengemisches, in dem das EE-Isomere überwiegt, in sehr guten Ausbeuten. Durch katalytische Hydrierung kann aus ihm in fast quantitativer Ausbeute (6R,7S,8R)-6,9-Dihydroxy-7,8-isopropylidendioxy-2,4-nonansäuremethylester hergestellt werden, der sich seinerseits beispielsweise nach dem Verfahren von Ohrui (vgl. Tetrahedron Lett. 1975, S. 3657) in 8 einfachen Stufen in (d)-Biotin überführen lässt. Somit gelingt die (d)-Biotinsynthese auf dem neuen Weg aus D-Arabinose in nur 11 Stufen und mit wesentlich besseren Ausbeuten als bisher.

Gegenüber anderen Syntheses von (d)-Biotin aus Kohlehydraten zeichnet sich dieser Weg durch Einsparung von Reaktionsschritten aus. So benötigen Ohrui et al. bei ihrem Verfahren aus D-Mannose oder D-Glucosamin [vgl. Tetrahedron Lett. 1975, S. 3657, bzw. Agr. Biol. Chem 42 (1978) S. 865], 16 Reaktionsstufen; Vogel et al. bei ihrem Verfahren aus D-Arabinose (vgl. Liebigs Ann. Chem. 1980, S. 1972) 15 Reaktionsstufen und Ogawa et al. bei ihrem Verfahren aus D-Glucose [vgl. Carbohydr. Res. 57 (1977), S. C31] 22 Stufen.

Beispiel 1

Herstellung von
(6R,7S,8R)-6,9-Dihydroxy-7,8-isopropylidendioxy-2,4-nonadiensäuremethyl-ester
in siedendem Toluol.

1,0 g (5,26 mmol) fein gepulverte und unter stark vermindertem Druck getrocknete 3,4-O-Isopropyliden-D-arabinose wurde bei Raumtemperatur in 150 ml absolutem Toluol gelöst. Dann wurde die Apparatur mit trockenem Stickstoff gefüllt und 2,08 g (578 mmol) [3-Carbomethoxypropen-(2)-yliden-(1)]-triphenylphosphoran zugefügt und durch Umschwenken suspendiert. Anschliessend wurde das Reaktionsgemisch aufgeheizt und unter langsam strömendem Stickstoff, der Rückflusskühler war mit einem aufgesetzten NaOH-Rohr versehen, gekocht. Nach 15 Minuten war mittels Dünnschichtchromatographie (DC) kein Ausgangszucker mehr nachweisbar (Laufmittel: reiner Essigsäureethylester). Nach 30-minütigem Kochen wurde auf 20 °C abgekühlt und die dunkle Lösung unter vermindertem Druck eingedampft. Es verblieben 3,22 g eines rotbraunen Öls als Rückstand. Dieser wurde in ca. 5 ml Essigsäureethylester aufgenommen, über eine kurze Kieselgelsäule (ø 6,5 cm, Länge 4,5 cm) mit Essigsäureethylester chromatographiert. Vorlauf 100 ml. Die folgenden 130 ml enthielten chromatographisch einheitlichen

(6R,7S,8R)-6,9-Dihydroxy-7,8-isopropylidendioxy-2,4-nonadiensäuremethylester

in Form eines E,E/E,Z-Gemisches (überwiegend E,E). Ausbeute: 1,17 g entsprechend 82% der Theorie.

Beispiel 2 und Vergleichsbeispiele

Analog Beispiel 1 wurden jeweils 1,0 g 3,4-O-Isopropyliden-D-arabinose mit 2,08 g 3-Carbomethoxy-propen-(2)-yliden-(1)-triphenylphosphoran

in einem Lösungsmittel umgesetzt. Die verwendeten Lösungsmittel, Lösungsmittelmengen, Reaktionstemperaturen, Reaktionszeiten und die erzielten Ausbeuten an

(6R,7S,8R)-6,9-Dihydroxy-7,8-isopropylidendioxy-2,4-nonadiensäuremethylester

sind in den Tabellen 1 und 2 zusammengestellt.

Tabelle 1

| Beisp. | Lösungsmittel | Temp. [°C] | Zeit [Min] | Ausbeute [%] |
|---|---|---|---|---|
| 2 | Toluol (140 ml) | 80 | 30 | 76 |

Tabelle 2

| Vergleichs-beispiele | Lösungsmittel | Temp. [°C] | Zeit [Min] | Ausbeute [%] |
|---|---|---|---|---|
| 3 | Benzol (150 ml) | Sieden (80) | 30 | 67 |
| 4 | Acetonitril | Sieden (82,6) | 30 | + |
| 5 | Ethanol | Sieden (79) | 20 | 25++ |
| 6 | Ethanol+++ | Sieden | 20 | 35++ |

\+ DC ergab ein so komplexes Gemisch, dass nicht aufgearbeitet wurde
++ starke Zersetzungserscheinungen
+++ in Gegenwart von 60 mg Benzoesäure durchgeführt

**Patentanspruch**

Verfahren zur Herstellung von

(6R,7S,8R)-6,9-Dihydroxy-7,8-isopropylidendioxy-2,4-nonadien-säurealkylestern

der allgemeinen Formel I

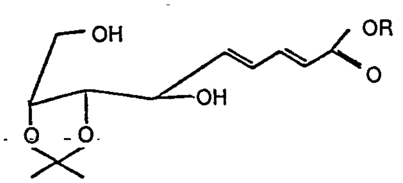

(I)

in der R für einen Alkylrest mit 1 bis 4 C-Atomen steht, durch Umsetzen von 3,4-O-Isopropyliden-D-arabinose der Formel II

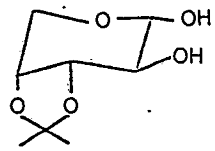

(II)

mit den Phosphoryliden der allgemeinen Formel III

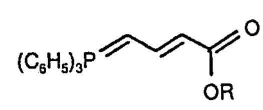

(III)

in der R die obige Bedeutung aufweist, in einem Lösungsmittel bei erhöhter Temperatur, dadurch gekennzeichnet, dass man die Umsetzung in Toluol bei Temperaturen von 80 bis 115 °C durchführt.

**Claim**

1. A process for the preparation of an alkyl (6R,7S,8R)-6,9-dihydroxy-7,8-isopropylidene-dioxy-2,4-nonadienoate

of the formula I

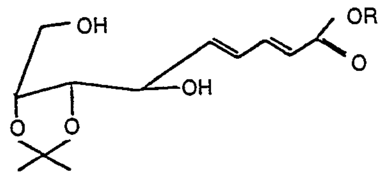

(I)

where R is alkyl of 1 to 4 carbon atoms, by reacting 3,4-O-isopropylidene-D-arabinose of the formula II

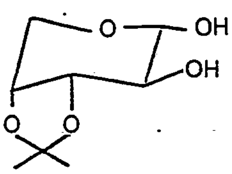

(II)

with a phosphorylide of the formula III

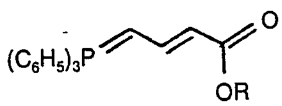

(III)

where R has the above meaning, in a solvent at elevated temperatures, wherein the reaction is carried out in toluene at from 80 to 115 °C.

**Revendication**

Procédé pour la préparation d'esters alkyliques de l'acide (6R,7S,8R)-6,9-dihydroxy-7,8-isopropylidène-dioxy-2,4-nonadiénoïque de formule générale I

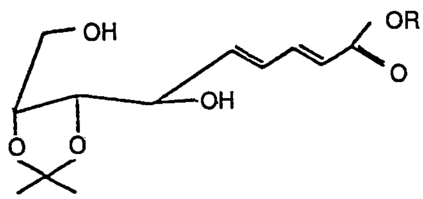

(I)

dans laquelle R est mis pour un radical alkyle à 1–4 atomes de C, par réaction de 3,4-O-isopropylidène-D-arabinose de formule II

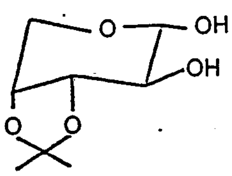

(II)

avec le phosphorylidène de formule générale III

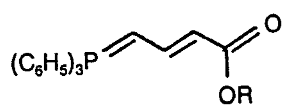

(III)

dans laquelle R présente la signification donnée ci-dessus, dans un solvant à température élevée, caractérisé en ce qu'on mène la réaction dans le toluène à des températures de 80 à 115 °C.